# EUROPÄISCHE PATENTSCHRIFT

(11) **EP 1 421 380 B1**
(45) Veröffentlichungstag und Bekanntmachung des Hinweises auf die Patenterteilung: **14.01.2009**
(21) Anmeldenummer: 02772172.9
(22) Anmeldetag: 20.08.2002
(51) Int. Cl.: G01N 33/487, C12M 1/34

(54) **VORRICHTUNG UND VERFAHREN ZUR ERFASSUNG BIOELEKTRISCHER SIGNALE AUS ELEKTROPHYSIOLOGISCH AKTIVEN BEREICHEN IN SPHÄROIDEN**
DEVICE AND METHOD FOR DETECTING BIOELECTRIC SIGNALS FROM ELECTROPHYSIOLOGICALLY ACTIVE REGIONS IN SPHEROIDS
DISPOSITIF ET PROCEDE POUR DETECTER DES SIGNAUX BIOELECTRIQUES A PARTIR DE ZONES ELECTROPHYSIOLOGIQUEMENT ACTIVES DE SPHEROIDES

(30) Priorität: 30.08.2001 DE 10142393
(43) Veröffentlichungstag der Anmeldung: 26.05.2004
(73) Patentinhaber: Fraunhofer-Gesellschaft zur Förderung der angewandten Forschung e.V., 80686 München (DE)
(72) Erfinder: THIELECKE, Hagen, 66440 Blieskastel (DE); ROBITZKI, Andrea, 68519 Viernheim (DE)
(74) Vertreter: Rösler, Uwe
(86) Internationale Anmeldenummer: PCT/EP2002/009267
(87) Internationale Veröffentlichungsnummer: WO 2003/020125

(56) Entgegenhaltungen:
- WO-A-01/25769
- DE-A- 19 946 458
- GB-A- 2 232 769
- MOLCKOVSKY A ET AL: "Monitoring of cell and tissue responses to photodynamic therapy by electrical impedance spectroscopy" PHYSICS IN MEDICINE AND BIOLOGY, APRIL 2001, IOP PUBLISHING, UK, Bd. 46, Nr. 4, Seiten 983-1002, XP002243462 ISSN: 0031-9155
- THIELECKE H ET AL: "A novel cell-positioning technique for extracellular recording and impedance measurement on single cells using planar electrode substrates" ENGINEERING IN MEDICINE AND BIOLOGY SOCIETY, 1998. PROCEEDINGS OF THE 20TH ANNUAL INTERNATIONAL CONFERENCE OF THE IEEE HONG KONG, CHINA 29 OCT.-1 NOV. 1998, PISCATAWAY, NJ, USA,IEEE, US, 29. Oktober 1998 (1998-10-29), Seiten 2872-2875, XP010320726 ISBN: 0-7803-5164-9

## Beschreibung

### Technisches Gebiet

Die Erfindung bezieht sich auf eine Vorrichtung sowie auf ein Verfahren zum Erfassen bioelektrischer Signale aus elektrophysiologisch aktiven Bereichen in Sphäroiden. Insbesondere wird dargelegt, wie die Wirkung pharmazeutischer vorzugsweise neuropharmakologischer oder neurotoxischer Wirkstoffe auf Sphäroide erfasst werden können ohne dabei die Sphäroide zu schädigen, so dass sie weiteren Untersuchungsmöglichkeiten zu Verfügung stehen können.

### Stand der Technik

Um die Wirkung von Substanzen, bspw. pharmakologische Wirkstoffe auf lebende Systeme routinemäßig erfassen zu können, sind in den letzten Jahren Biosensoren entwickelt worden, die auf lebenden Zellen basieren, siehe hierzu Bousse, L.: "Whole Cell Biosensors", Sensors and Actuators Band 34, 270-275 (1996). Derartige, auf biologischen Zellen basierende Biosensoren weisen hauptsächlich Monolayer-Zellkulturen als biologisches Erkennungssystem auf, doch können Wirkstoffverursachte komplexe Zell/Zell- oder Zell/Matrix-Interaktionen durch derartige Monolayer-Zellkulturen oft nicht mit der gewünschten Genauigkeit und Zuverlässigkeit technisch erfasst werden. Hinzukommt, dass die Wirkung von Neuropharmaka oder Umwelttoxine eben gerade jene komplexe Zell/Zell-Interaktionen im Zentralen Nervensystem zur Folge haben, die es gilt messtechnisch zu erfassen, um weitere Einblicke in die biochemische Reaktionskette derartiger Substanzen auf biologisches Zellmaterial zu erhalten. Schließlich weisen die auf Monolayer-Zellkulturen basierenden Biosensoren den Nachteil auf, dass die mit diesen Sensoren erhältlichen Messergebnisse nur über einen beschränkten Aussagegehalt über das tatsächliche Reaktionsvermögen biologischer Zellen bspw. auf einen gezielten Wirkstoffeintrag, verfügen, zumal die Monolayer-Zellkulturen in dieser Form in der belebten Natur nicht vorkommen.

Um diesen Nachteil zu vermeiden ist man bei der Untersuchung derartiger Substanzen vielmehr auf biologische Modelle angewiesen, die der *in vivo* Situation hinsichtlich der interzellulären sowie intrazellulären Interaktionen möglichst nahe kommen. Dreidimensionale Zellsysteme spiegeln die *in vivo* Situation wesentlich besser wider als einzelne Zellen oder Monolayer-Zellkulturen. Somit ist es notwendig, zum Test von Wirkstoffen, die auf die Beeinflussung der Zell/Zell-Interaktionen zielen, dreidimensionale Zellsysteme zu verwenden.

Um die neuropharmakologischen oder neurotoxischen Wirkung von Substanzen bspw. außerhalb von Tiermodellen zu Testen, werden in an sich bekannter Weise bioelektrische Signale von *ex vivo* Gewebeschnitten mit Hilfe von Glasmikroelektroden oder Nadelelektroden abgeleitet. Zur Aufzeichnung von Signalverläufen mittels Multikanalableitungen werden planare Elektrodenanordnungen, sogenannte Multielektrodenarrays eingesetzt. Jedoch müssen *ex vivo* Gewebeschnitte aufwendig aus Tiermodellen präpariert werden, sind nicht standardisierbar und auf die vorhandenen Tiermodelle beschränkt. Da überdies *ex vivo* Gewebeschnitte schnell degenerieren, sind Gewebeschnitte für Langzeituntersuchungen nicht geeignet. Dies jedoch wäre bei der Untersuchung von Neuropharmaka oder Umwelttoxine und ihr Einfluss auf biologische Gewebe von äußerst hoher Relevanz.

Ein interessantes Untersuchungsobjekt für die vorstehend aufgeworfene Fragestellung sind sogenannte Sphäroide, die als kugelförmige Zellaggregate aufgefasst werden können. Aus der Literatur sind bspw. Untersuchungen der Retinogenese und der Retinaregeneration bekannt, bei denen unter konstanten Bedingungen derartige regenerierte kugelförmige Zellaggregate, sogenannte Retinosphäroide, gewonnen werden (siehe hierzu Moscona, A. A.: "Development of Heterotypic Combination of Dissociated Embryonic Chick Cells". Proc. Soc. Exp. Bio. Med 292, 410-416 (1956); Vollmer, G., Layer, P. G., Gierer, A.: "Reaggregation of Embryonic Chick Retina Cells: Pigment Epithelial Cells Induce a High Order of Stratification". Neurosci. Lett. 48, 191-196 (1984)). Diese werden durch geeignete Kultivierung von dissoziierten Zellen aus embryonalen Retinae reaggregiert.

Molckovsky und Wilson (Phys. Med. Biol. 46 (2991), p.983-1002) verwendeten in ihren Impedanz-Untersuchungen von Sphäroiden eine wannenförmnige Messkammer, die in Längsrichtung zwei gegenüberliegende Elektroden umfasste, die durch ihre Stirnseite den Raum für die Positionierung des Sphäroids definierten.

Die WO 01/25769 andererseits offenbart eine Array-artige Vorrichtung zur parallelen Impedanzmessung an einer Vielzahl von Zellen, wobei die einzelnen Messkammern topfartige Form aufweisen und durch Silizium-Ätztechnologie hergestellt werden. Die Elektroden sind hierbei in der Wand um am Boden der jeweiligen Messzelle lokalisiert.

In der DE 199 46 458.8 wird hierzu eine Vorrichtung sowie ein Verfahren zur Charakterisierung von Sphäroiden mittels Impedanzspektroskopie beschrieben. Damit kann der Einfluss von Substanzen auf die Proliferation, Morphologie und Membraneigenschaften der *in vitro* Gewebe, d.h. außerhalb des lebenden Organismus, bestimmt werden. Ortsaufgelöste Informationen aus dem Inneren des Sphäroids können jedoch mit der bekannten Methode nicht gewonnen werden. Auch ist es mit der in vorstehender Druckschrift beschriebenen Vorrichtung nicht möglich Informationen über intrazelluräre elektrische Potentiale in Form sogenannter bioelektrischer Signale zu erhalten, an Hand derer die Wirkung pharmazeutischer Wirkstoffe, insbesondere von neuropharmakologischen oder neurotoxischen Wirkstoffen erfasst werden kann.

### Darstellung der Erfindung

Es besteht die Aufgabe eine Vorrichtung sowie ein Verfahren zum Erfassen bioelektrischer Signale aus Sphäroiden derart anzugeben, dass es möglich ist die neurotoxische und neuropharmakologische Wirkung von Substanzen auf biologisches Material im Wege einer in vitro Untersuchung möglichst nahe an der in vivo Situation hinsichtlich der interzellulären sowie intrazellulären Interaktionen zu erfassen.

Die Lösung der der Erfindung zugrundeliegenden Aufgabe ist im Anspruch 1 angegeben. Gegenstand des Anspruches 14 ist ein erfindungsgemäßes Verfahren. Den Erfindungsgedanken vorteilhaft weiterbildende Merkmale sind Gegenstand der Unteransprüche sowie der Beschreibung unter Bezugnahme auf die Ausführungsbeispiele zu entnehmen.

Erfindungsgemäß weist die Vorrichtung zum Erfassen bioelektrischer Signale aus Sphäroiden die nachfolgenden Komponenten auf:
- Eine Messkammer mit einer Messkammerwand, die in Art einer zylindrisch Kapillare oder in Topfform ausgebildet ist, aus elektrisch nicht leitendem Material besteht und zumindest in einem Messbereich einen Innenquerschnitt aufweist, der dem größten Querschnitt eines Sphäroids entspricht.
   Vorzugsweise ist die Messkammer als Kapillare ausgebildet, mit Kapillarwänden und einem Kapillarboden, die den Messbereich für das Sphäroid definieren. Die Querschnittsgröße des von den Kapillarwänden eingeschlossenen Messbereiches ist derart gewählt, dass das Sphäroid längs seines größten Umfangsrandes in mechanischen Kontakt zur Messkammer- bzw. Kapillarwand steht, so dass das Sphäroid innerhalb des Messbereiches eine möglichst feste Raumlage einnimmt, die für die weitere Messung des Sphäroids von großem Vorteil ist. Um die Positionierung des Sphäroids innerhalb der Messkammer bzw. Kapillare weiter zu verbessern ist in einer bevorzugten Ausführungsform der Vorrichtung im Kapillarboden eine Unterdruckleitung angeschlossen, um das Sphäroid mittels Saugwirkung regelrecht in innerhalb des Messbereiches zu fixieren.
- Eine vielzahl von Elektroden sind in einer gemeinsamen Ebene innerhalb der Messkammerwand angeordnet, wobei die Elektroden jeweils eine, zum Messbereich orientierte frei zugängliche Elektrodenoberfläche aufweisen. Die Elektroden sind vorzugsweise in jener Ebene innerhalb der Messkammerwand angeordnet in der das Sphäroid mit seinem größten Umfangsrand die Messkammerwand berührt. Die vorstehende Forderung, dass die Elektroden in einer Ebene angeordnet sind, ist nicht notwendigerweise mathematisch exakt zu verstehen, d.h. im Sinne längs einer, der Messkammerinnenwand umlaufenden gedachten Linie. Die Elektroden sollten zumindest mit ihren zum Messbereich orientierten Elektrodenoberflächen längs des Kontaktbereiches zwischen Sphäroid und Messkammerwand angeordnet sein, so dass es mit einer an die einzelnen Elektroden angeschlossenen Impedanzmessanordnung möglich ist die Impedanzverteilung ortsaufgelöst in der durch die Elektrodenanordnung vorgegebenen Schnittebene innerhalb des Sphäroids zu ermitteln. Hierbei wird über die einzelnen Elektroden innerhalb des Sphäroids ein elektrischer Strom induziert und die über dem Sphäroid abfallende elektrische Spannung gemessen. Aus Strom und Spannung wird die Impedanz gebildet. Zur Durchführung eines sogenannten Impedanzimaging wird die Frequenz des in den Sphäroid induzierten Stroms über einen zusammenhängenden Frequenzbereich variiert und die sich dabei ergebende Impedanz als Funktion der Frequenz aufgezeichnet. So ist es mit Hilfe eines derartigen Impedanzimaging-Systems möglich aus der aufgezeichneten Impedanzverteilung bei verschiedenen Frequenzen die Gewebeparameter innerhalb der Schnittebene ortsaufgelöst zu ermitteln. Man erhält auf diese Weise Kenntnis über den inneren Aufbau des Sphäroids innerhalb der Schnittebene. So zeichnen sich sogenannte elektrophysiologisch aktive Bereiche durch eine in der Konsistenz kompakte Untereinheit aus, die sich von übrigen nicht organisierten Bereichen innerhalb des Sphäroids im Impedanzverhalten zu unterscheiden vermögen. Eben gerade jene elektrophysiologisch aktiven Bereiche sind von großem Interesse bei der Fragestellung der Auswirkung bestimmter Wirkstoffe auf biologische Zellen, zumal in diesen Bereichen als eine Art Zellantwort auf das Einwirken eines Wirkstoffes auf die jeweilige Zelle technisch erfassbare und auswertbare Signale erzeugt werden.
   So verfügen elektrophysiologisch aktive Bereiche innerhalb des Sphäroids über eine bioelektrische Aktivität, durch die das elektrische Oberflächenpotenzialverhalten des gesamten Sphäroids beeinflusst wird. Ändert sich bspw. durch Einwirken einer bestimmten Substanz auf das Sphäroid und damit zugleich auf die elektrophysiologisch aktiven Bereiche dessen bioelektrische Aktivität, so wirkt sich dies direkt auf das Oberflächenpotenzial des Sphäroids aus. Vorzugsweise mit Hilfe eines Potenzialableitungssystem, das mit den um das Sphäroid angeordneten Elektroden verbunden ist, ist man in der Lage Oberflächenpotenziale längs der Schnittebene durch das Sphäroid zu erfassen und letztendlich eine Information über die bioloelektrische Aktivität der innerhalb der Schnittebene befindlichen elektrophysiologisch aktiven Bereiche zu erhalten.

Sowohl für die Impedanzmessung als auch für das Erfassen der Oberflächenpotenziale müssen die freien Elektrodenoberflächen nicht notwendigerweise in unmittelbaren Kontakt zur Oberfläche des Sphäroids stehen. Vielmehr dient auch eine innerhalb der Messkammer eingebrachte Kulturflüssigkeit, die bspw. das Nährmedium darstellt innerhalb der das Sphäroid gezüchtet wird, als elektrisch leitendes Medium, durch das ein elektrischer Kontakt zwischen den Elektroden und der Oberfläche des Sphäroids herstellbar ist.

In einer einfachen Ausführungsform schließen die freien Elektrodenoberflächen bündig mit der Innenwand der Messkammer ab, so dass ein direkter Kontakt zwischen den Elektrodenoberflächen und dem Sphäroid vorherrscht.

In einer alternativen Ausführungsform befinden sich die Elektroden derart innerhalb sogenannter Verbindungskammern, die einseitig offen in die Messkammer münden, dass die freien Elektrodenoberflächen von der Messkammerinnenwand zurückversetzt sind. Dies hat zunächst den Vorteil, dass die Elektroden leichter austauschbar bzw. auswechselbar sind und überdies können bei geeigneter geometrische Ausbildung und Anordnung der Verbindungskammern, bspw. zur Messkammer konisch zulaufend, größere freie Elektrodenoberflächen eingesetzt werden. In Hinblick auf eine möglichst geringe Phasengrenzimpedanz ist der Einsatz möglichst großer Elektrodenflächen wünschenswert, die durch entsprechende beabstandete Anordnung innerhalb konisch ausgebildeter Verbindungskammern von der Messkammerinnenwand realisierbar sind. Wie bereits vorstehend erwähnt dient die Kulturflüssigkeit, die zusammen mit dem Sphäroid in der Messkammer eingebracht ist, als elektrisches Kontaktmedium zwischen den Elektroden und der Sphäroidoberfläche.

Insbesondere in Hinblick auf die Untersuchung von Sphäroiden im industriellen Massstab, um die Wirkungsweise neuer pharmakologischer Wirkstoffe zu testen, eignen sich Halbleitermaterialien für den Aufbau der vorstehend beschriebenen Vorrichtung. Mit den Mitteln der Halbleitertechnik lassen sich eine Vielzahl arrayförmig angeordneter Messkammern realisieren, die in Form und Größe für Untersuchung von Sphäroiden angepasst sind und erlauben somit eine statistische Auswertung aufgrund einer großen Anzahl untersuchter Sphäroide. Ein konkretes Ausführungsbeispiel hierzu wird unter Bezugnahme der im weiteren beschriebenen Figuren genauer dargelegt.

Mit Hilfe der vorstehenden Vorrichtung lassen sich Sphäroide, ohne sie zu zerstören, auf ihre bioelektrische Aktivität hin untersuchen, um sie nachfolgend schadlos zur weiteren Beobachtung in ein Kulturmedium zurückzuführen. So ist es möglich ein und dasselbe Sphäroid in zeitlichen Abständen mehrmals zu vermessen, um etwaige Wirkstoff-bedingte Degradationserscheinungen feststellen zu können. Hierdurch können nach Auswertung einer Vielzahl derartiger Sphäroide, die innerhalb eines Kulturmediums zusätzlich einem bestimmten Wirkstoff ausgesetzt sind, statistische Aussagen über die Wirkungsweise von Wirkstoffen getroffen werden.

Im besonderen zeichnet sich das erfindungsgemäße Verfahren zur Erfassung bioelektrischer Signale aus Sphäroide durch die Kombination folgender Verfahrensschritte aus: Bereitstellen einer Vorrichtung der vorstehend beschriebenen Art, Einbringen und Positionieren eines Sphäroids innerhalb der Messkammer sowie Durchführen einer Impedanzmessung nach der Impedanzimaging-Methode zur ortsaufgelösten Bestimmung elektrophysiologisch aktiver Bereichen in dem Sphäroid. Um die bioelektrische Aktivität bestimmen zu können wird zusätzlich eine Oberflächenpotenzialmessung längs der durch die Elektrodenanordnung vorgegebenen Schnittebene durchgeführt.

Mit Hilfe des erfindungsgemäßen Verfahrens kann zum einen die Morphologie von multizellulären Sphäroiden ortsaufgelöst und nicht invasiv bestimmt und zudem können die Erregungsverläufe von elektrophysiologisch aktiven Bereichen in Sphäroiden präzise ermittelt werden. Insbesondere ermöglicht das Verfahren, dass die Wirkung von Substanzen bzw. Wirkstoffen auf 3D *in vitro* Modelle des zentralen Nervensystems non invasiv erfasst werden kann. Durch die vorstehend erläuterte Vorrichtung im Sinne eines Biosensorsystems können Langzeituntersuchungen der neurotoxischen und neuropharmakologischen Wirkung von Substanzen realisiert werden. Das biologische Erkennungselement eingesetzte Sphäroid wird nur für einen kurzen Zeitraum während der Impedanzmessung und Potenzialableitung in die Messkammer positioniert und kann unabhängig von der Messanordnung unter physiologischen Bedingungen kultiviert werden. Eine Adhäsion des Sphäroids wird durch die Gegenwart der Kulturflüssigkeit innerhalb der Messkammer weitgehend verhindert und unerwünschte Zell/Material-Wechselwirkungen werden minimiert. Je nach Fragestellung können somit für das Biosensorsystem Sphäroide oder 3D biologische Erkennungselemente mit unterschiedlichen Zelltypen in unterschiedlichen Lagen erzeugt werden.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnung exemplarisch beschrieben. Es zeigen:
- Fig. 1: Schematische Flussdiagrammdarstellung zur Durchführung des Analyseverfahrens,
- Fig. 2 a,b: Darstellungen einer Messkammer mit Sphäroid,
- Fig. 3 a,b,c: Querschnittsdarstellungen durch ein Sphäroid sowie Potentialbildaufnahme,
- Fig. 4: Querschnitt durch eine arrayförmige Messanordnung in Halbleitertechnologie,
- Fig. 5: Messanordnung,
- Fig. 6: Querschnitt durch eine Messkammer sowie
- Fig. 7: Querschnitt durch eine alternative Messkammer.

### Wege zur Ausführung der Erfindung, gewerbliche Verwendbarkeit

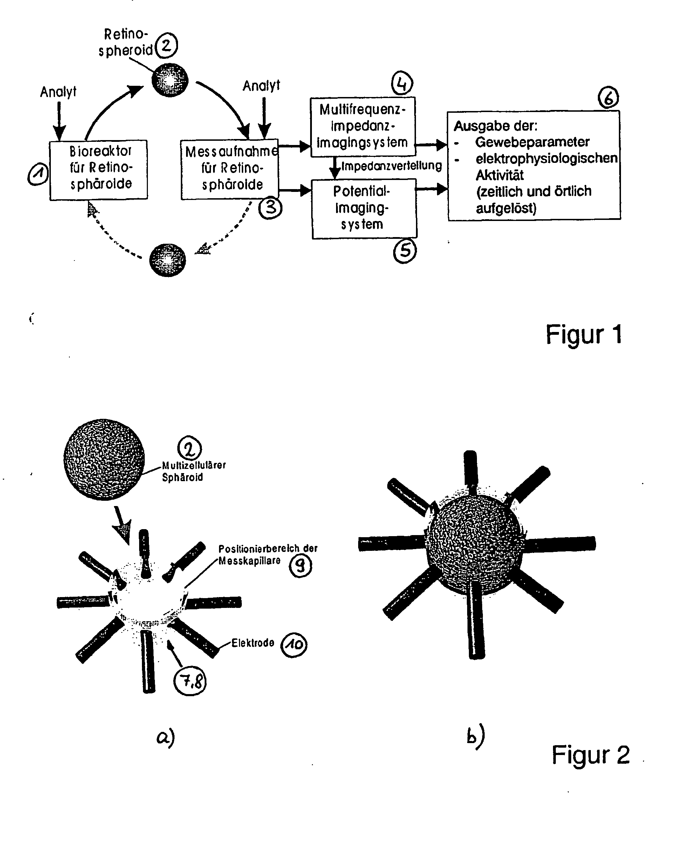

Am Beispiel der Untersuchung von reaggregierten Retinospheroiden soll das erfindungsgemäße Verfahren unter Bezugnahme auf Figur 1 erläutert werden:

Dissoziiert embryonale Zellen des zentralen Nervensystems werden in einem Bioreaktor 1 unter Mikrogravitationsbedingungen zu kugelförmigen neuronalen Reaggregationskulturen den sogenannten Retinospäroiden reaggregiert. Durch Zugabe von geeigneten Wachstumsfaktoren und/oder durch geeignete genetische Manipulationen wird erreicht, dass sich elektrophysiologisch aktive Zellbereiche im Sphäroid gleich verteilt ausbilden. Somit befindet sich in einer beliebigen Schnittebene, die durch das Zentrum des Sphäroids verläuft, mit hoher Wahrscheinlichkeit mindestens ein elektrophysiologisch aktiver Bereich.

Um die Wirkung einer Substanz auf die Sphäroide zu testen, ist wenigstens ein Sphäroid 2 aus dem Bioreaktor 1 zu isolieren und in Messkammer des Biosensorsystem 3 zu verbringen, um es dort als *in vitro* Modell zu testen. Sowohl im Bioreaktor 1 als auch in der Messkammer des Biosensors 3 befindet sich das Sphäroid 2 in einer Kulturflüssigkeit bzw. Analyt, so dass das Sphäroid beliebig zwischen dem Bioreaktor und der Messkammer ohne dabei Schaden zu erleiden austauschbar ist.

Mittels Multifrequenz-Impedanzimaging 4 wird die Lage und Ausdehnung der verschiedenen Zellbereiche in einer durch die Elektrodenanordnung innerhalb der Messkammer vorgegebenen Querschnittebene bestimmt, und anschließend durch elektrisches Quellenimaging 5 die bioelektrische Aktivität der einzelnen Zellbereichen in der Schnittebene ermittelt. Systeme und Algorithmen für das Impedanzimaging und das elektrische Quellenimaging sind grundsätzliche aus der medizinischen Tomographie bekannt, siehe Webster, J. G.: "Electrical Impedance Tomography". Adam Hilger, Bristol (1990).

Als Parameter 6 für die Wirkung von Substanzen auf das *in vitro* Gewebemodell dienen Änderungen der elektrophysiologischen Aktivität von bestimmten Bereichen im Sphäroid, die Korrelation der elektrophysiologischen Aktivität unterschiedlicher Bereiche sowie die Änderung von Gewebeparametern.

Zur Durchführung des Impedanzimaging und der Potentialableitung wird ein elektrophysiologisch aktiver Sphäroid 2 gemäß Figur 2 im gewünschtem Kulturstadium in eine Messkammer 7 positioniert. Je nach Fragestellung, wie z. B. Langzeituntersuchungen oder dynamische Stimulation, wird die zu prüfende Substanz der Kulturflüssigkeit im Bioreaktor oder der Kulturflüssigkeit in der Messkammer 7 zugegeben. Die Messkammer 7 wird vorzugsweise durch eine Kapillare 8 gebildet, die im Positionierbereich zylindrisch ausgebildet ist und deren Wand 9 aus elektrisch isolierendem Material besteht. Im Positionierbereich der Kapillare 8 sind in der Kapillarwand 9 in mindestens einer senkrecht zur Längsachse stehenden Ebene eine Vielzahl von Elektroden 10 angeordnet. Da die Elektroden 10 mit ihren freien Elektrodenoberflächen in dem in Figur 2 a, b gezeigten Ausführungsbeispiel bündig zur Messkammerinnenwand angeordnet sind, berühren sie den im Inneren der Messkammer eingebrachten Sphäroid 2 (Fig. 2 b) längs seines größten Umfanges in einer Schnittebene. Die Elektroden sind einzeln von außen zur Ansteuerung elektrisch kontaktiert (nicht dargestellt).

Diese Anordnung dient sowohl zur ortsaufgelösten Bestimmung der passiven elektrischen Eigenschaften des *in vitro* Gewebes als auch zur Bestimmung des räumlichen und zeitlichen Verlaufs der elektrophysiologischen Erregung.

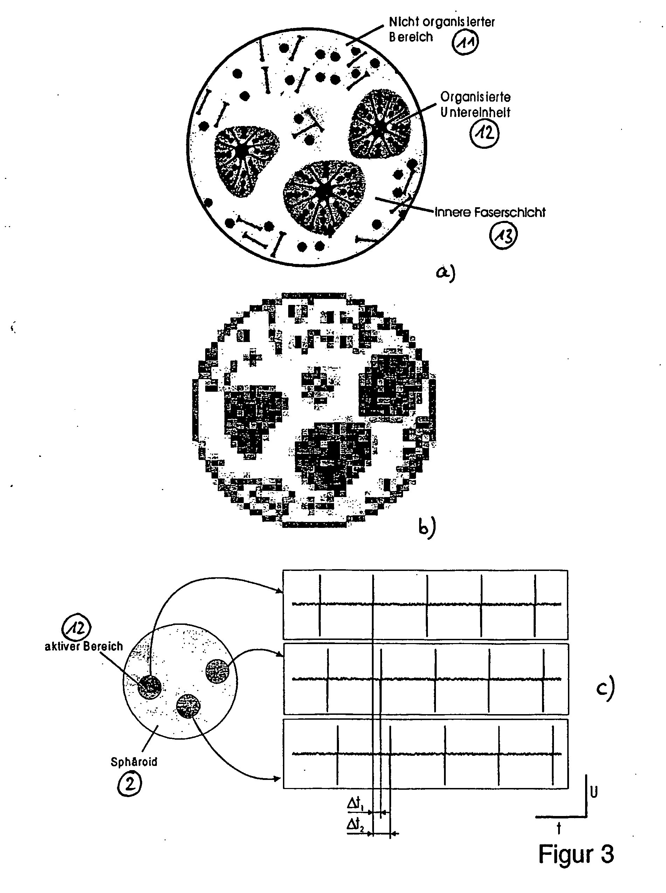

Zur Ermittlung der Impedanzverteilung der Schnittebene des Sphäroids, in der die Elektroden liegen, werden die Elektroden in geeigneter Weise mit einem Impedanzimaging-System verbunden. Aus den Impedanzverteilungen bei verschiedenen Frequenzen werden die Gewebeparameter ortsaufgelöst ermittelt. Hierzu ist in Figur 3 a ein tatsächlicher Schnitt durch ein Sphäroid dargestellt, der typischerweise nicht weiter organisierte Bereiche 11, organisierte Unterbereiche, die sogenannten elektrophysiologisch aktiven Bereiche 12 sowie innere Faserschichten 13 aufweist. In Figur 3b ist ein mittels Impedanzimaging ermitteltes Schnittbild dargestellt, das dem in Figur 3a tatsächlich wiedergegebenen Schnittbild entspricht. Die bioelektrische Aktivität bestimmter Bereiche wird aus den Oberflächenpotentialen, die mit den Elektroden abgeleitet werden, sowie aus der Impedanzverteilung bestimmt, siehe die Diagrammdarstellung in Figur 3c. Bei der Ableitung der Oberflächenpotentiale sind die Elektroden der Messkapillare mit einem Ableitsystem verbunden. Markante auswertbare Messsignale stellen insbesondere die an der Sphäroidoberfläche wahrnehmbaren Spannungspeaks (siehe Diagrammdarstellung) sowie deren zeitliche Aufeinanderfolge (Δt₁, Δt₂) dar. Eben jene Messgrößen werden durch die Gegenwart bestimmter Wirkstoffe nachweislich beeinflusst, wodurch Aussagen über die Wirkung bestimmter Substanzen auf biologisches Material getroffen werden können.

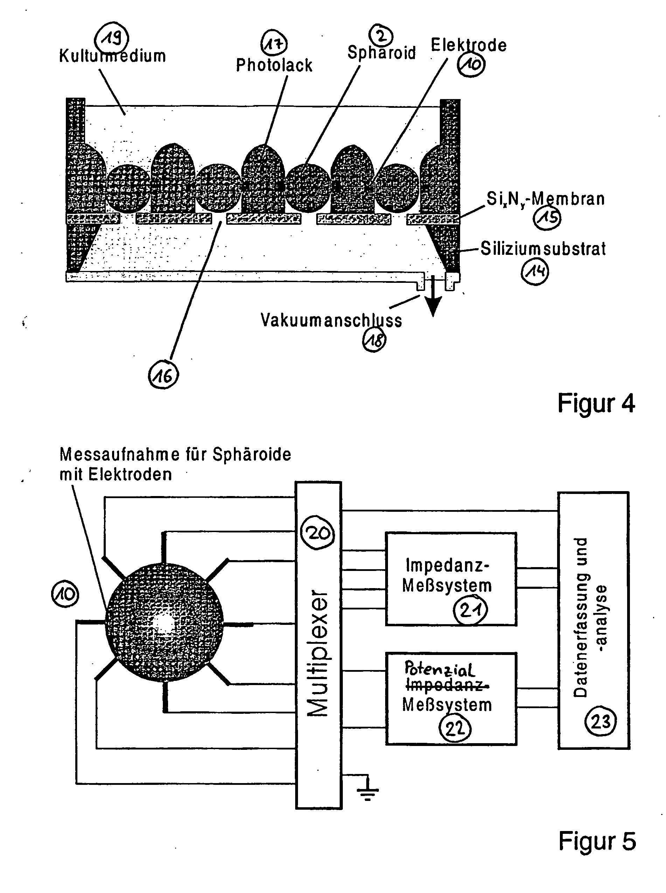

In Figur 4 ist eine Messkammeranordnung dargestellt, bei der auf einem planaren Substrat 14 in einer Array-Struktur eine Vielzahl einzelner Messkammern 7 angeordnet sind. Zur Herstellung einer derartigen Messkammeranordnung wird auf einem Siliziumsubstrat 14 eine Siliziumnitridschicht 15 mit ca.1 µm Dicke abgeschieden. Die Siliziumnitridschicht 15 wird als Membranen von ihrer Unterseite freigelegt. Ferner werden Mikrolöcher 16 mit 20 µm Durchmesser in die Membrane 15 durch Trockenätzen eingebracht. Anschließend wird ein Photolack 17 mit einer Dicke von 40 µm aufgebracht. Im Photolack 17 werden konzentrisch zu den Mikrolöchern zylinderförmige Messkammern 7 (Durchmesser 150 µm) freigeätzt. Auf dem Photolack 17 wird eine Metallschicht in einer Dicke von 10 µm abgeschieden und so strukturiert, dass die Messkammern 7 von acht kreisförmig angeordneten, äquidistant beabstandeten Elektroden 10 umgeben sind. Anschließend wird erneut eine Photolackschicht 17 (50 µm) abgeschieden und strukturiert.

Um die Sphäroide beim Einbringen in die einzelnen Messkammern 7 nicht zu schädigen, werden die Kanten der Messkammern 7 abgerundet. Damit ein Unterdruck 18 zur Positionierung der Sphäroide angelegt werden kann, wird die gefertigte Mikrostruktur auf eine Platte mit Bohrung und Schlauchanschluss geklebt. Zur Durchführung einer Messung wird der gesamte Bereich der Messkammer 7 mit einer Kulturflüssigkeit 19 gefüllt, um Adhäsionseffekte zwischen den einzelnen Sphäroiden und der Messkammerwand zu vermeiden.

Die Elektroden 10 die in der Messkammer eingebracht sind gemäß Figur 5 über einen Multiplexer 20 mit einem Impedanzmesssystem 21 und mit einem Potenzialableitsystem 22 verbunden. Die Messdaten werden an eine Datenerfassung- und Analyseeinheit 23 übertragen, die auch den Multiplexer 20 steuert.

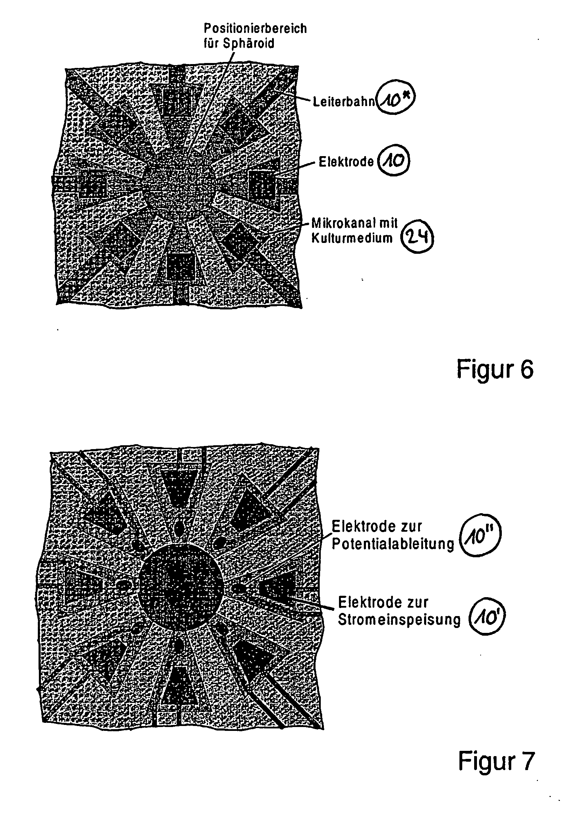

Gemäß Ausführungsbeispiel in Figur 6, die einen Querschnitt durch eine Messkammer 7 zeigt, sind sternförmig um die Messkammer 7 konisch zulaufende Verbindungskammern 24 angeordnet, die in die Messkammer einmünden. Dadurch kann die Größe der Metallelektroden 10, die mit jeweils durch Leiterbahnen 10* kontaktiert sind, unabhängig von der Größe der Messkammer gewählt werden, und die Phasengrenzimpedanz der Elektroden 10 lässt sich durch größere Elektrodenflächen verringern.

In einem weiteren Ausführungsbeispiel gemäß Figur 7 sind zur Realisierung von Impedanzmessungen in einer Vier-Elektroden-Anordnung in jedem Kanal eine Elektrode 10' zur Stromeinspeisung und eine zur Potentialableitung 10" angeordnet.

### Bezugszeichenliste

- 1: Bioreaktor
- 2: Sphäroid
- 3: Biosensor, Messanordnung
- 4: Impedanzmessanordnung
- 5: Potenzialableitungsanordnung
- 6: Auswerteparameter
- 7: Messkammer
- 8: Kapillare
- 9: Messkammerwand
- 10: Elektrode
- 10*: Leiterbahn
- 10': Elektrode zur Stromeinspeisung
- 10": Elektrode zur Potenzialableitung
- 11: Nicht organisierter Bereich
- 12: Organisierter Bereich, elektrophysiologischer Bereich
- 13: Innere Faserschicht
- 14: Substrat
- 15: Membran
- 16: Mikroloch
- 17: Photolack
- 18: Unterdruckanschluss
- 19: Kulturflüssigkeit
- 20: Multiplexer
- 21: Impedanzsystem
- 22: Potenzialbleitungssystem
- 23: Datenerfassungs- und analysesystem
- 24: Verbindungskammer

## Patentansprüche

1. Vorrichtung zum Erfassen bioelektrischer Signale aus Sphäroiden,
**dadurch gekennzeichnet, dass** eine in Art einer zylindrischen Kapillare oder in Topfform ausgebildete Messkammer mit einer Messkammerwand vorgesehen ist, die aus einem elektrisch nicht leitenden Material besteht,
dass die Messkammer zumindest einen von der Messkammerwand umfassten Innenquerschnitt aufweist, der einem Messbereich entspricht und derart bemessen ist, dass ein Sphäroid längs seines gesamten größten Umfangsrandes in mechanischen Kontakt zur Messkammerwand bringbar ist,
dass eine Vielzahl von Elektroden in einer gemeinsamen Ebene innerhalb der Messkammerwand angeordnet ist, in der das Sphäroid mit der Messkammerwand in Kontakt bringbar ist,
dass die Elektroden jeweils eine, zum Messbereich orientierte frei zugängliche Elektrodenoberfläche aufweisen, und
dass eine Impedanzmessanordnung vorgesehen ist, die mit den Elektroden verbunden ist.

2. Vorrichtung nach Anspruch 1,
**dadurch gekennzeichnet, dass** ein Potenzialableitungssystem mit den Elektroden verbunden ist.

3. Vorrichtung nach Anspruch 1 oder 2,
**dadurch gekennzeichnet, dass** die in Art einer zylindrischen Kapillare ausgebildete Messkammer die Vielzahl von Elektroden in einer Ebene orthogonal zur Längserstreckung der Kapillare vorsieht.

4. Vorrichtung nach einem der Ansprüche 1 bis 3,
**dadurch gekennzeichnet, dass** die frei zugänglichen Elektrodenoberflächen der Elektroden bündig mit der Messkammerinnenwand ausgebildet sind.

5. Vorrichtung nach einem der Ansprüche 1 bis 3,
**dadurch gekennzeichnet, dass** innerhalb der Messkammerwand eine Anzahl von Verbindungskammern vorgesehen sind, die mit dem Messbereich offen verbunden sind und in einer gemeinsamen Ebene, im Umfangsrichtung um den Messbereich gleich verteilt angeordnet sind, und
dass innerhalb der Verbindungskammern jeweils eine Elektrode eingebracht ist, deren
zum Messbereich orientierte frei zugängliche Elektrodenoberfläche von der Messkammerinnenwand beabstandet ist.

6. Vorrichtung nach Anspruch 5,
**dadurch gekennzeichnet, dass** innerhalb der Verbindungskammem jeweils eine zweite Elektrode vorgesehen ist, die mit dem Potenzialableitungssystem verbunden ist.

7. Vorrichtung nach einem der Ansprüche 1 bis 6,
**dadurch gekennzeichnet, dass** die Messkammer mit einer elektrisch leitenden Flüssigkeit befüllbar ist.

8. Vorrichtung nach einem der Ansprüche 1 bis 7,
**dadurch gekennzeichnet, dass** mit der Messkammer eine Unterdruckleitung verbunden ist.

9. Vorrichtung nach einem der Ansprüche 1 bis 8,
**dadurch gekennzeichnet, dass** die topfartig ausgebildete Messkammer am Topfboden eine Unterdruckleitung vorsieht, zur Positionierung und Fixierung eines in die topfartige Messkammer eingebrachten Sphäroids mittels Unterdruck.

10. Vorrichtung nach einem der Ansprüche 1 bis 9,
**dadurch gekennzeichnet, dass** die Impedanzmessanordnung sowie das Potenzialableitungssystem über einen Multiplexer mit den Elektroden verbunden sind.

11. Vorrichtung nach einem der Ansprüche 1 bis 10,
**dadurch gekennzeichnet, dass** eine Datenerfassungs- und Datenauswerteeinheit mit der Impedanzmessanordnung sowie dem Potenzialableitungssystem verbunden ist.

12. Vorrichtung nach einem der Ansprüche 1 bis 11,
**dadurch gekennzeichnet, dass** eine Vielzahl von Messkammern arrayförmig angeordnet und in planarer Halbleitersubstrattechnik ausgebildet ist.

13. Vorrichtung nach einem der Ansprüche 1 bis 12,
**dadurch gekennzeichnet, dass** die Elektroden in Umfangsrichtung der Messkammerwand gleich verteilt angeordnet sind.

14. Verfahren zur Erfassung bioelektrischer Signale aus Sphäroide durch die Kombination folgender Verfahrensschritte:
- Bereitstellen einer Vorrichtung nach einem der Ansprüche 1 bis 13,
- Einbringen und Positionieren eines Sphäroids innerhalb der Messkammer
- Durchführen einer Impedanzmessung nach der Impedanzimaging-Methode zur ortsaufgelösten Bestimmung elektrophysiologisch aktiver Bereichen in dem Sphäroid.

15. Verfahren nach Anspruch 14,
**dadurch gekennzeichnet, dass** eine Oberflächenpotenzialmessung zur Erfassung der bioelektrischen Aktivität durchgeführt wird.

16. Verfahren nach Anspruch 14 oder 15,
**dadurch gekennzeichnet, dass** die Impedanzmessung bei unterschiedlichen Anregungsfrequenzen durchgeführt wird, um ein Impedanzspektrum zu erhalten.

17. Verwendung der Vorrichtung nach einem der Ansprüche 1 bis 13 zur Untersuchung der Wirkung von Wirkstoffen auf Sphäroide.

18. Verwendung nach Anspruch 17,
**dadurch gekennzeichnet, dass** die Wirkstoffe pharmazeutische Wirkstoffe , insbesondere neuropharamakologische oder neurotoxische Substanzen sind.

19. Verwendung nach Anspruch 17 oder 18,
**dadurch gekennzeichnet, dass** in einem ersten Schritt ein mit einem Wirkstoff versetzter Sphäroid aus einem Kulturmedium entnommen und in die Messkammer der Vorrichtung eingebracht wird,
in einem darauffolgenden Schritt wird die Impedanzmessung und/oder die Potenzialableitung non invasiv am Sphäroid durchgeführt und
in einem letzen Schritt wird das Sphäroid in das Kulturmedium schadlos zurück verbracht.

## Claims

1. A device for detecting bioelectric signals from spheroids wherein
- a measuring chamber designed as a cylindrical capillary or pot-shaped having a measuring chamber wall which is composed of a non-electrically conducting material,
- the measuring chamber has at least one inner cross section enclosed by the measuring wall, the inner cross section which corresponds to the measuring region and is dimensioned in such a manner that a spheroid can be brought along its entire largest circumferential edge in mechanical contact with the measuring chamber wall,
- a multiplicity of electrodes is disposed in a common plane within the measuring chamber wall in which the spheroid can be placed in contact with the measuring chamber wall,
- the electrodes each have an electrode surface which is oriented freely accessible toward the measuring region, and
- an impedance measuring arrangement is provided which is connected to the electrodes.

2. A device according to claim 1,
wherein a potential determining system is connected to the electrodes.

3. A device according to claim 1 or 2,
wherein the measuring chamber which is designed as a cylindrical capillary provides a multiplicity of electrodes in one plane which is disposed orthogonally to the length of the capillary.

4. A devices according to one of the claims 1 to 3,
wherein the freely accessible electrode surfaces of the electrodes are designed flush with the inner wall of the measuring chamber.

5. A device according to one of the claims 1 or 3,
wherein provided in the measuring chamber wall is a number of connecting chambers which are openly connected to the measuring region and are disposed evenly distributed in a common plane in circumferential direction around the measuring region, and
wherein placed inside each of the connecting chambers is an electrode,
whose freely accessible electrode surface which is oriented towards the measuring region is spaced at a distance from the inner wall of the measuring chamber.

6. A device according to claim 5,
wherein a second electrode is is provided inside each of the connecting chambers and is connected to the potential determining system.

7. A device according to one of the claims 1 to 6,
wherein the measuring chamber can be filled with an electrically conducting liquid.

8. A device according to one of the claims 1 to 7,
wherein a partial vacuum conduit is connected to the measuring chamber.

9. A device according to one of the claims 1 to 8,
wherein the bottom of the pot-shaped measuring chamber is provided with a partial vacuum conduit for positioning and fixing a spheroid placed in the pot-shaped measuring chamber by means of a partial vacuum.

10. A device according to one of the claims 1 to 9,
wherein the impedance measuring arrangement and the potential determining system are connected to the electrodes via a multiplexer.

11. A device according to one of the claims 1 to 10,
wherein a data collection and data evaluation unit is connected to the impedance measuring arrangement and to the potential determining system.

12. A device according to one of the claims 1 or 11,
wherein a multiplicity of measuring chambers is disposed in an array-like manner and is designed in a planar semiconductor substrate technology.

13. A device according to one of the claims 1 to 12,
wherein the electrodes are disposed evenly distributed in circumferential direction of the measuring chamber wall.

14. A method for detecting bioelectric signals from spheroids with the combination of the following steps:
- providing of a device according to claims 1 to 13,
- placing and positioning of a spheroid inside the measuring chamber,
- conducting an impedance measurement according to the impedance-imaging method for locally resolved determination of electrophysiologically active regions in the spheroid.

15. The method according to claim 14,
wherein a surface potential measurement is conducted to detect the bioelectric activity.

16. The method according to claim 14 tor 15,
wherein the impedance measurement is conducted at different triggering frequencies in order to obtain an impedance spectrum..

17. Use of the device according to one of the claims 1 or 13 to study the effect of substances on spheroids.

18. Use according to claim 17,
wherein the substances are pharmaceutical substances, in particular, neuropharamacological or neurotoxic substances.

19. Use according to claim 17 or 18,
wherein in a first step, a spheroid to which a substance has been applied is removed from a culture medium and is placed in the measuring chamber of the device,
in a subsequent step, the impedance measurement and/or the potential determination is conducted non-invasively on the spheroid, and in a last step the spheroid is returned unharmed to the culture medium.

## Revendications

1. Dispositif d'enregistrement de signaux bioélectriques de sphéroïdes,
**caractérisé en ce qu'**il est prévu une chambre de mesure réalisée à la manière d'un capillaire cylindrique ou en forme de cuvette avec une paroi de chambre de mesure qui est composée d'un matériau non conducteur électrique,
que la chambre de mesure comporte au moins une section transversale interne entourée par la paroi de chambre de mesure et correspondant à une zone de mesure et est dimensionnée de manière à ce qu'un sphéroïde puisse être amené en contact mécanique avec la paroi de la chambre de mesure le long de tout son plus grand bord périphérique, qu'une multitude d'électrodes sont disposées dans un plan commun dans la paroi de chambre de mesure dans laquelle le sphéroïde peut être amené en contact avec la paroi de chambre de mesure,
que les électrodes présentent respectivement une surface d'électrode librement accessible orientée vers la zone de mesure et
qu'il est prévu un dispositif de mesure d'impédance qui est relié aux électrodes.

2. Dispositif selon la revendication 1,
**caractérisé en ce qu'**un système de dérivation de potentiel est relié aux électrodes.

3. Dispositif selon la revendication 1 ou 2,
**caractérisé en ce que,** dans la chambre de mesure réalisée à la manière d'un capillaire cylindrique, la multitude d'électrodes sont disposées dans un plan orthogonal à l'extension longitudinale du capillaire.

4. Dispositif selon une des revendications 1 à 3,
**caractérisé en ce que** les surfaces d'électrode librement accessibles des électrodes sont réalisées à fleur avec la paroi interne de la chambre de mesure.

5. Dispositif selon une des revendications 1 à 3,
**caractérisé en ce que**, dans la paroi interne de la chambre de mesure, il est prévu un certain nombre de chambres de liaison qui sont reliées ouvertement à la zone de mesure et sont disposées dans un plan commun, réparties régulièrement autour de la zone de mesure dans le sens circonférentiel, et
que, dans les chambres de liaison, est installée respectivement une électrode
dont la surface d'électrode librement accessible orientée vers la zone de mesure est espacée de la paroi interne de la chambre de mesure.

6. Dispositif selon la revendication 5,
**caractérisé en ce que,** dans les chambres de liaison, il est prévu respectivement une deuxième électrode qui est reliée au système de dérivation de potentiel.

7. Dispositif selon une des revendications 1 à 6,
**caractérisé en ce que** la chambre de mesure peut être remplie d'un liquide conducteur électrique.

8. Dispositif selon une des revendications 1 à 7,
**caractérisé en ce qu'**une conduite en dépression est reliée à la chambre de mesure.

9. Dispositif selon une des revendications 1 à 8,
**caractérisé en ce qu'**il est prévu dans la chambre de mesure réalisée en forme de cuvette, au fond de la cuvette, une conduite en dépression pour le positionnement et la fixation par dépression d'un sphéroïde installée dans la chambre de mesure en forme de cuvette.

10. Dispositif selon une des revendications 1 à 9,
**caractérisé en ce que** le dispositif de mesure d'impédance de même que le système de dérivation de potentiel sont reliés aux électrodes par un multiplexeur.

11. Dispositif selon une des revendications 1 à 10,
**caractérisé en ce qu'**une unité d'enregistrement et d'exploitation de données est reliée au dispositif de mesure d'impédance de même qu'au système de dérivation de potentiel.

12. Dispositif selon une des revendications 1 à 11,
**caractérisé en ce qu'**une multitude de chambres de mesure sont disposées en forme de réseau et suivant une technique planaire de substrat à semi-conducteurs.

13. Dispositif selon une des revendications 1 à 12,
**caractérisé en ce que** les électrodes sont disposées réparties régulièrement dans le sens circonférentiel de la paroi de la chambre de mesure.

14. Procédé d'enregistrement de signaux bioélectriques de sphéroïdes par la combinaison des étapes opératoires suivantes :
- élaboration d'un dispositif selon une des revendications 1 à 13,
- introduction et positionnement d'un sphéroïde dans la chambre de mesure,
- réalisation d'une mesure d'impédance suivant le procédé d'imagerie d'impédance pour la détermination définie par localisation de zones électrophysiologiquement actives dans le sphéroïde.

15. Procédé selon la revendication 14,
**caractérisé en ce qu'**on réalise une mesure de potentiel de surface pour enregistrer l'activité bioélectrique.

16. Procédé selon la revendication 14 ou 15,
**caractérisé en ce que** la mesure d'impédance est réalisée à différentes fréquences d'excitation pour obtenir un spectre d'impédance.

17. Utilisation du dispositif selon une des revendications 1 à 13 pour l'analyse de l'effet des substances actives sur les sphéroïdes.

18. Utilisation selon la revendication 17,
**caractérisé en ce que** les substances actives sont des substances actives pharmaceutiques, notamment des substances neuropharmacologiques ou neurotoxiques.

19. Utilisation selon la revendication 17 ou 18,
**caractérisé en ce que,** au cours d'une première étape, un sphéroïde transformé avec une substance active est prélevé dans un milieu de culture et introduit dans la chambre de mesure du dispositif,
au cours d'une étape suivante, la mesure d'impédance et/ou la dérivation de potentiel sont réalisées de manière non invasive au niveau du sphéroïde et,
au cours d'une dernière étape, le sphéroïde est remis sans dommages dans le milieu de culture.
